# EUROPEAN PATENT APPLICATION

(11) **EP 4 792 769 A1**
(43) Date of publication of application: **19.08.2026**
(21) Application number: 25157778.9
(22) Date of filing: 13.02.2025
(51) Int. Cl.: A61B 34/30, A61B 34/32, A61F 9/007

(54) **SURGICAL ROBOTIC SYSTEM FOR SUBRETINAL INJECTION**

(71) Applicant: Carl Zeiss Meditec AG, 07745 Jena (DE)
(72) Inventor: MEENINK, Hildebert Christiaan Matthijs, 5656 AG Eindhoven (NL); SMIT, Jorrit, 5656 AG Eindhoven (NL); DA COL, Tommaso, 5656 AG Eindhoven (NL); AARTS, Elke, 5656 AG Eindhoven (NL); CERFONTEIJN, Esmee, 5656 AG Eindhoven (NL)
(74) Representative: DeltaPatents B.V.

(57) **Abstract**

A surgical robotic system for intraocular procedures is provided. The system comprises a surgical arm having a movable arm part, wherein the movable arm part comprises an end effector configured to hold a surgical instrument, the surgical instrument comprising a needle 121 for subretinal injection of a substance below a retinal surface 220. The system further comprises an actuator subsystem configured to actuate the movable arm part and a processor subsystem configured to control the actuator subsystem to control a longitudinal positioning of the surgical instrument during the intraocular procedure. The processor subsystem is configured to control the actuator subsystem to perform at least part of a subretinal injection procedure by indenting the retinal surface 220 with a tip 122 of the needle 121 and, from the indented state of the retinal surface 220, performing a piercing movement by advancing the needle 121 longitudinally to penetrate the retinal surface 220 and reach a target depth 123 below the retinal surface 220. A corresponding computer-implemented method for controlling such a surgical robotic system is also provided.

## Description

### TECHNICAL FIELD

The presently disclosed subject matter relates to a surgical robotic system for use in an intraocular procedure, to a computer-implemented method for controlling the surgical robotic system during the intraocular procedure, and to a computer program comprising instructions which, when executed by a processor, cause the processor to carry out the method.

### BACKGROUND

The need to inject fluids into the subretinal space has become important for treating various retinal diseases, such as macular degeneration, diabetic retinopathy, and inherited retinal disorders. Subretinal injections allow delivery of therapeutic agents, such as drugs, gene therapies, or stem cells, directly to specific layers of the retina, which may improve treatment effectiveness while reducing unwanted side effects. Also, non-therapeutic fluids may be injected, such as imaging dyes or contrast agents, for example for diagnostic purposes or to assist in surgical procedures. Furthermore, other non-fluid substances, such as hydrogels, may also be delivered to the subretinal space in advanced therapeutic or diagnostic applications.

The retina is thin, ranging from approximately 100 µm in the central foveal region to 250 in other regions, and in rare instances, reaching up to 400 µm. In certain pathological conditions, the retina may be weakened and exhibit a reduced thickness. This thinness of the retina, both in typical and pathological case, necessitates a high level of precision during subretinal injections to mitigate the risk of complications.

Surgical instruments used for such procedures, including subretinal injection (SRI) needles, are designed to be highly precise to meet this need. SRI needles, which are instruments designed to penetrate tissues and deliver small volumes of fluids, are commonly used for subretinal injections. For example, in treating certain diseases, an SRI needle may be used to inject a small volume of a therapeutic agent, such as 10 µL, into the retina. Nevertheless, if the needle is not positioned correctly, the therapeutic agent may flow back into the vitreous cavity, potentially causing an inflammation reaction which may necessitate follow-up treatment or additional surgical procedures, such as flushing. Furthermore, the therapeutic effect may be compromised if an excessive amount of the therapeutic agent flows back, as insufficient agent may reach the intended target area. This issue could also lead to the preventive use of a larger quantity of the therapeutic agent, thereby increasing production requirements and overall costs. In cases where a syringe and needle system with multiple chambers is employed to administer more than one therapeutic agent, precise administration of each agent becomes even more critical to ensure the intended therapeutic effects are achieved without compromising efficacy or causing unintended side effects. Conversely, if the needle penetrates too deeply, it may damage the Bruch's membrane, which is an important immunological barrier of the eye. To avoid or at least reduce these risks, precise control of the needle tip is required to ensure that the needle tip penetrates the retinal surface accurately, the needle tip is positioned at the correct subretinal depth, and that the fluid is delivered (injected) in a controlled manner.

It is known to use OCT feedback to confirm whether the needle is at the correct position for injection. For example, in US20190110682 A1, the use of an optical coherence tomography (OCT) sensor is introduced in a vitreoretinal surgery application in order to detect when a surgical injector penetrates a desired tissue layer of the eye.

Despite these advancements, performing subretinal injections with precision and control remains challenging when done manually. Surgical robotic systems have been introduced in the general field of surgery, including intraocular surgery, to enhance precision, steadiness, and control compared to handheld instruments. A surgical robotic system may, for example, include a surgical arm comprising a movable arm part, wherein the movable arm part comprises an end effector for holding a surgical instrument. The surgical arm may position the instrument based on instructions provided either by a human operator or through automated control. A human-machine interface may allow the operator to input commands to move the instrument, while an actuator in the robotic system moves the arm accordingly. In tele-operated systems, a human operator may control a master device, such as a motion controller, which transmits movement instructions to the surgical arm acting as a slave device. Alternatively, the system may be configured to perform at least part of an intraocular procedure automatically, without requiring direct input from the human operator.

Nevertheless, while surgical robotic systems can perform or assist in intraocular procedures in general, there remains a need for a surgical robotic system specifically designed to improve the performance of subretinal injections.

### SUMMARY

In a first aspect of the presently disclosed subject matter, a surgical robotic system is provided for use in an intraocular procedure, comprising:
- a surgical arm comprising a movable arm part, wherein the movable arm part comprises an end effector for holding a surgical instrument, wherein the surgical instrument comprises a needle for subretinal injection of a substance below a retinal surface;
   - an actuator subsystem configured to actuate the movable arm part;
   - a processor subsystem configured to control the actuator subsystem to control a longitudinal positioning of the surgical instrument during the intraocular procedure, wherein the processor subsystem is configured to control the actuator subsystem to perform at least part of a subretinal injection procedure by:
   - with a tip of the needle, indent the retinal surface; and
   - from said indented state of the retinal surface, perform a piercing movement, wherein the piercing movement comprises advancing the surgical instrument longitudinally by a forward distance to penetrate the retinal surface and reach a target depth below the retinal surface.

In a further aspect of the presently disclosed subject matter, a computer-implemented method is provided for controlling a surgical robotic system during an intraocular procedure,
wherein the surgical robotic system comprises:
- a surgical arm comprising a movable arm part, wherein the movable arm part comprises an end effector for holding a surgical instrument, wherein the surgical instrument comprises a needle for subretinal injection of a substance below a retinal surface;
- an actuator subsystem configured to actuate the movable arm part;
wherein the method comprises:
- controlling the actuator subsystem to control a longitudinal positioning of the surgical instrument during the intraocular procedure,
wherein said control of the actuator subsystem comprises performing at least part of a subretinal injection procedure by:
- with a tip of the needle, indenting the retinal surface; and
- from said indented state of the retinal surface, performing a piercing movement, wherein the piercing movement comprises advancing the surgical instrument longitudinally by a forward distance to penetrate the retinal surface and reach a target depth below the retinal surface.

In a further aspect of the presently disclosed subject matter, a computer program is provided comprising instructions which, when executed by a processor, cause the processor to carry out the method as described above. The computer program may for example be stored on a non-transitory computer-readable medium.

The above aspects of the presently disclosed subject matter involve providing a surgical robotic system that includes a surgical arm comprising a movable arm part. The movable arm part may comprise an end effector for holding and manipulating a surgical instrument. The combination of the movable arm part and the end effector may also be referred to as an instrument manipulator. For example, the end effector may comprise an instrument connector for mounting the surgical instrument, e.g., in a removable manner. The surgical instrument may be configured to enter a patient's eye, for example through a trocar. In the present case, the surgical instrument comprises, or may be, a needle for subretinal injection of a substance below a retinal surface, e.g., an SRI needle. The substance may include a therapeutic agent, such as a drug or gene therapy, or a non-therapeutic substance, such as a dye or contrast agent. In some examples, the substance may be a fluid or a hydrogel.

The surgical instrument, e.g., the needle, may typically have a longitudinal axis that passes through a tip of the needle. The movable arm part may typically have multiple degrees of freedom (DOF), which may allow the tip of the needle to move longitudinally (e.g., to advance towards or retract away from a surgical target on the retinal surface) and laterally along a plane perpendicular to the longitudinal axis (e.g., along the retinal surface). In general, the movable arm part may have sufficient DOFs to enable adjustment of the pose of the surgical instrument, where the term 'pose' may refer to the position and orientation of the instrument. Thereby, the position and angle of approach of the needle tip with respect to the retinal surface may be controlled.

An actuator subsystem may be provided for actuating the movable arm part to adjust the pose of the surgical instrument. For this purpose, the actuator subsystem may include one or more actuators that may be configured to directly actuate the movable arm part and/or the end effector, or to actuate components through which the instrument manipulator may be movably suspended, such as a linkage system or a base. Using the actuator(s), the pose of the surgical instrument may be adjusted, for example to perform longitudinal movement, lateral movement, and orientation adjustments. The actuator subsystem may also be referred to as a driving mechanism.

The surgical robotic system may further comprise a processor subsystem, such as a control unit comprising one or more microprocessors, associated memory, communication interfaces, and input/output circuitry. The processor subsystem may generally be configured to control the actuator subsystem to adjust the longitudinal position, and as mentioned before, typically also the lateral position and orientation, of the surgical instrument during an intraocular procedure. The processor subsystem may operate under control of a human operator, for example directly (e.g., in a tele-operated manner), indirectly, or, in some examples, may perform surgical steps automatically.

Specifically, the processor subsystem may be configured to control the actuator subsystem to perform at least part of a subretinal injection procedure. This may mean that certain steps of the subretinal injection procedure may be carried out automatically by the surgical robotic system, without requiring the human operator (also referred to as 'user') to directly steer or manually control the robot during those steps. Nevertheless, it will be appreciated that the human operator may remain involved in the procedure, for example by providing confirmation between individual surgical steps, as also elucidated with reference to optional features and embodiments.

The processor subsystem may, as one of the automatic steps, indent the retinal surface with the tip of the instrument at the location where the subretinal injection is to be performed. The location may specifically be the intended entry point for piercing or penetration. In this context, 'indentation' may refer to a controlled deformation or depression of the retinal surface caused by the instrument tip. For example, the indentation may be in the range of 20-150 µm relative to the surrounding retinal surface. The indentation depth may be chosen depending on the local thickness of the retina. For example, for a local retina thickness of approximately 150 µm, the indentation depth may be chosen within the range of 20 - 50 µm, whereas higher indentation depths, such as 150 µm, may be chosen for a local retina thickness of approximately 400 µm. In some cases, the indentation depth may be selected as a percentage of the thickness of the retina, for example any value in the range of 5%-30% of the retina thickness, preferably 10%-20%. Examples of specific indentation percentages include, but are not limited, to 5%, 10%, 15%, 20%, 25%, 30%, etc.

The indentation may be performed as an automatic step, for example by longitudinally advancing the needle through controlled operation of the actuator subsystem. As a subsequent automatic step, the processor subsystem may control the actuator subsystem to perform a piercing movement. The piercing movement may include, but is not necessarily limited to, advancing the surgical instrument longitudinally by a forward distance sufficient to penetrate the retinal surface and reach a target depth below the retinal surface. This movement may be carried out automatically, for example without requiring direct user steerage or manual control.

The inventors have recognized that starting from an indented state, rather than, for example, positioning the needle in front of the surface, results in improved penetration. This improved penetration has been demonstrated in ex-vivo and in-vivo experiments. The improvement may arise from the restoration force of the indented state, which may provide a reactive 'back force' against the advancing needle. This back force may result from the elastic properties of the retinal tissue, where the indentation causes a buildup of elastic potential energy, similar to a compressed spring. When the needle is advanced longitudinally, the elastic recoil force of the tissue may assist in overcoming the resistance of the retinal surface, enabling smoother and more controlled penetration. Additionally, the relative velocity of the needle tip with respect to the tissue may be effectively increased, further aiding penetration. Starting from the indented state also provides a well-defined and controlled starting point for penetration. From this starting point, the penetration depth may be precisely determined based on the forward distance travelled by the needle. Achieving such a controlled and consistent indentation manually, particularly at the required magnitude (e.g., 20-150 µm), may be challenging. Furthermore, manually controlling the forward distance of the penetrating movement is also difficult to perform with the necessary precision, especially when a relatively high velocity is desired to overcome the resistance of the surface. Advantageously, by automating these specific steps, the surgical robotic system may achieve precise and accurate penetration of the retinal surface to a target depth.

The following optional features are described with reference to the surgical robotic system but correspond to method steps in the computer-implemented method and corresponding instructions in the computer program.

Optionally, the processor subsystem is configured to control the actuator subsystem to indent the retinal surface by:
- positioning the tip of the needle to touch the retinal surface;
- accessing intraoperative sensor data to detect and confirm the touch of the retinal surface; and
- upon confirmation of the touch, advancing the needle longitudinally by an indentation distance, for example 20-150 µm, to achieve the indented state of the retinal surface.

By first positioning the tip of the needle to touch the retinal surface and confirming the touch using intraoperative sensor data, such as data from a contact sensor mounted on the surgical instrument or intraoperative OCT sensor data, a correct starting position may be established for the indentation. This may ensure that the indentation is performed within the desired range, for example, the aforementioned 20-150 µm. The specific indentation depth may be determined dynamically by the processor subsystem based on the retinal thickness at or near the penetration site. For example, the processor subsystem may analyse the intraoperative sensor data to determine the local thickness of the retina at or near the penetration site. Based on this analysis, the processor subsystem may calculate the indentation depth, for example relative to the retinal thickness, and in a specific example, as a percentage. In other examples, the indentation depth may be predetermined or provided as user input by a user. From the confirmed touching state of the needle, the needle may then be advanced in a controlled manner to achieve the indented state of the retinal surface.

Optionally, the processor subsystem is further configured to confirm the indented state of the retinal surface before initiating the piercing movement, wherein said confirmation comprises receiving a user input indicative of the indented state or analysing intraoperative sensor data indicative of said indentation of the retinal surface.

Indentation may be important for penetration performance, and it may be desirable to verify the indented state before initiating the piercing movement, e.g., whether the retinal surface is indented by a desired amount. Accordingly, the surgical robotic system may confirm the indentation, for example by receiving input from the user, such as directly confirming the indentation or indirectly by indicating whether to proceed or abort, or by analysing intraoperative sensor data. For example, OCT sensor data may visually represent the indentation, and the indentation distance may be determined using image analysis techniques, for example using heuristically designed image analysis techniques or using machine learning. This may allow the system to verify whether the desired indentation has been achieved, and if not, it may be corrected or at least ensure that penetration does not proceed under incorrect conditions.

Optionally, the processor subsystem is further configured to:
- obtain an assessment of a quality of penetration of the retinal surface after the piercing movement; and
- based on the assessment, either continue or abort the subretinal injection procedure, wherein continuing comprises controlling the surgical instrument to perform the subretinal injection of the substance via the needle.

Improper penetration of the retinal surface, particularly in terms of reaching the target depth or successfully completing the piercing movement, may pose significant risks during the injection process. For example, if the needle does not penetrate deeply enough, or does not penetrate correctly, the injected substance may flow back into the vitreous cavity, potentially causing inflammation. Conversely, if the needle penetrates too deeply, the substance may be injected behind the immunological barrier of the eye (e.g., the Bruch's membrane), which may cause additional complications. It is noted that this optional aspect of the presently disclosed subject matter may also be independently applied from the indenting of the retinal surface and the performing of the piercing movement, in that a surgical robotic system may be provided of which the processor subsystem is configured to perform these optional steps but without being configured to indenting of retinal surface and perform the piercing movement. To avoid or reduce the chance of such complications, the surgical robotic system may confirm the quality of penetration before proceeding with the substance injection. This assessment may rely on one or more metrics, such as whether the target depth has been reached. Such assessment may for example be carried out by analysing intraoperative sensor data, or by obtaining data from an encoder which is indicative of the degree of advancement of the needle. Only if the quality of penetration is confirmed to meet the desired requirements, for example by ensuring that one or more metrics exceed predefined thresholds, the system may continue with the subretinal injection, for example by controlling a pump which is configured to deliver the substance via the needle through a pump control interface. This may ensure that the substance is not injected unless the target site has been properly reached, reducing the risk of incorrect substance delivery.

Optionally, the surgical robotic system further comprises a sensor interface configured to access intraoperative sensor data indicative of a shape of the retinal surface after penetration, and the processor subsystem is further configured to assess the quality of penetration by:
- analysing a shape of the retinal surface at a penetration site based on the intraoperative sensor data; and
- determining that the quality of penetration is unsatisfactory if the retinal surface exhibits an indentation at the penetration site which exceeds a predefined indentation threshold.

The inventors have recognized from experiments that the presence of an indentation at the penetration site may serve as an important indicator of incorrect penetration which can lead to reflux, where the injected substance flows back into the vitreous cavity. Accordingly, the surgical robotic system may assess the quality of penetration by analysing the shape of the retinal surface at the penetration site using intraoperative sensor data, such as OCT sensor data. The degree of indentation may be determined, and if the indentation exceeds a predefined threshold, or in general is measurable when it should not be, the quality of penetration may be deemed unsatisfactory. In such cases, the surgical robotic system may prevent continuation of the subretinal injection procedure, for example by aborting the procedure entirely or by initiating a reattempt of the penetration. Conversely, if no measurable indentation is present, or the degree of indentation remains below a predefined threshold, the quality of penetration may be considered satisfactory, at least with respect to minimizing the risk of substance backflow into the vitreous cavity, and the surgical robotic system may continue with the subretinal injection, for example by controlling a pump which is configured to deliver the substance via the needle through a pump control interface.

Optionally, the assessment of the quality of penetration is obtained at least in part through user input indicative of an evaluation of the penetration. In addition, or as an alternative, to assessing the quality of penetration from intraoperative sensor data, the assessment of the quality of penetration may be obtained at least in part through user input indicative of an evaluation of the penetration. A user (e.g., the human operator) may confirm that the quality of penetration is sufficient or indicate that the quality is insufficient. If used in conjunction with automatic assessment, user input may serve as an override, for example to abort or redo the penetration despite a positive automatic assessment. This involvement of the user may provide an additional layer of verification, enhancing the safety and reliability of the injection procedure.

Optionally, the surgical robotic system further comprises a pump control interface operatively connected to a pump configured to deliver the substance for injection via the needle, and the processor subsystem is further configured to control the pump to regulate an injection volume during the subretinal injection procedure, for example by gradually increasing a volumetric flow rate. The surgical robotic system may further automate part of the injection procedure by automating the step of delivering the substance, namely by controlling a pump, for example via a pump control interface operatively connected to the pump. This may enable controlled delivery of the substance, allowing precise regulation of the injection process. For example, the processor subsystem may control the pump to gradually increase the volumetric flow rate during the subretinal injection procedure. A gradual increase in flow rate has been found to reduce the risk of reflux compared to sudden steps in volumetric flow. Such gradual control may be difficult to achieve manually or, at least may require cognitive effort from the user, potentially distracting from monitoring other critical aspects of the injection procedure.

Optionally, the processor subsystem is further configured to:
- monitor the injection volume based on intraoperative sensor data; and
- stop the injection of substance when a target injection volume has been delivered.

The target injection volume may not be fixed or preset but may instead depend on the bleb size, which refers to the substance-filled area created below the retinal surface during the injection. The bleb size may be monitored using intraoperative sensor data, such as OCT sensor data, and the system may automatically stop the injection when the desired bleb size or corresponding volume is reached. The volume may be estimated by determining a surface area of the bleb.

Optionally, the processor subsystem is further configured to retract the needle in a stepwise manner after the subretinal injection of the substance, for example in increments of 100 µm. The inventors have recognized that by allowing the needle to retract gradually, potential damage to the retinal pigment epithelium (RPE) caused by the injection jet may be reduced, as the distance between the needle tip and the RPE increases, reducing the jet's impact. Furthermore, retracting the needle in smaller incremental steps may help avoid the formation of a donut-shaped bleb caused by resistance along the needle shaft, instead promoting a more uniform and natural bleb shape. Additionally, controlled stepwise retraction supports a smoother withdrawal from the retinotomy, allowing the cut in the retina to settle and close more effectively, potentially reducing the risk of complications and promoting better healing.

Optionally, the processor subsystem is further configured to pause after the subretinal injection of the substance and, following the pause, initiate the stepwise retraction of the needle. The pause following the subretinal injection, and optionally before the stepwise retraction of the needle, may fulfil several functions. For example, pause may allow the bleb to relax, promoting settlement of the surrounding tissue and enabling pressure equalization between the bleb and the intraocular environment. Pressure equalization may also reduce the likelihood of reflux of the injected substance, as a balanced pressure may minimize the tendency of the substance to escape. Additionally, the pause may stabilize the injection system, including the pump and substance conduit (e.g., injection tubing), preventing or reducing reflux from the instrument tip into the vitreous cavity during or after needle retraction.

Optionally, the processor subsystem is configured to perform the piercing movement by first retracting the surgical instrument longitudinally by a retraction distance, and after the retracting, advancing the surgical instrument longitudinally by a forward distance which exceeds the retraction distance to penetrate the retinal surface and reach the target depth below the retinal surface. First retracting the surgical instrument may allow the indentation to partially restore due to the elastic properties of the retinal tissue. Advancing the surgical instrument during the time when the indentation is restoring may improve penetration because the restoring tissue exerts a reactive force against the needle, which can assist in overcoming the resistance of the surface. Additionally, the relative motion of the needle tip with respect to the tissue may increase during the restoration phase, which may also improve penetration.

Optionally, the retraction distance and the forward distance are jointly selected to achieve the target depth of 150-250 µm below the retinal surface. The retraction distance and forward distance may be jointly selected to achieve a target depth of 150-250 µm below the retinal surface to facilitate precise needle positioning within the retina. For example, to reach such a target depth, the needle may need to travel 200-400 µm below the retinal surface in case of a relatively shallow angle of approach. The target depth may generally be selected to penetrate through the entire thickness of the retina up to the retinal pigment epithelium (RPE) layer in order to align with the general objective of subretinal injections, which may involve the needle opening being positioned beneath the retinal surface to achieve optimal substance delivery. In some examples, the target depth may be selected dynamically by the processor subsystem, for example based on intraoperative sensor data, to account for variations in retinal thickness and the angle of approach to ensure that detachment may involve the deepest retinal layer along with the upper surface of the RPE layer.

Optionally, the processor subsystem is further configured to determine the target depth intraoperatively based on an angle of approach of the needle and an estimate of a thickness of the retina. While the target depth may be predetermined, e.g., preoperatively, it may also be dynamically determined by the surgical robotic system based on intraoperative conditions, such as the thickness of the retina. Since injections should not be made at too shallow a depth to avoid reflux, yet must remain within the retinal layers to prevent damage to the Bruch's membrane, the estimate of the thickness of the retina may allow selecting an appropriate target depth. For example, the estimate of the thickness of the retina may be obtained by analysing intraoperative sensor data, e.g., OCT sensor data. The angle of approach of the needle may also influence the required forward distance to reach the target depth. Accordingly, the processor subsystem may take the angle of approach into account when determining the forward distance for the advancement of the needle.

Optionally, the needle comprises a bevelled tip with an outer diameter below 100 µm measured at an onset of the bevel. This tip design may facilitate cutting through the retinal surface layer, thereby improving penetration performance and reducing damage to the retinal surface. For example, the outer diameter may be 80 - 160 µm, while the bevel of the tip may be bevelled at an angle of 45° ± 10°, preferably 45° ± 5°, more preferably 45° ± 2.5°. The tip of the needle may be made out of a translucent plastic, preferably a translucent thermoplastic, more preferably a translucent polymer such as polyimide. Such translucent plastics may be partially translucent to OCT, which can aid in monitoring various steps of the injection process, such as determining the target depth and assessing piercing performance.

It will be appreciated by those skilled in the art that two or more of the above-mentioned embodiments, implementations, and/or aspects of the presently disclosed subject matter may be combined in any way deemed useful.

Modifications and variations of the surgical robotic system, computer-implemented method, and computer program, in line with the described features, variations, and optional aspects of each respective entity or activity, may be carried out by a person skilled in the art based on the present description.

### BRIEF DESCRIPTION OF THE DRAWINGS

These and other aspects of the invention are apparent from and will be elucidated with reference to the embodiments described hereinafter. In the drawings,
Fig. 1 shows a schematic representation of a surgical robotic system;
Fig. 2 illustrates the degrees of freedom (DoF) of a surgical instrument mounted to a surgical robotic system of the type shown in Fig. 1;
Fig. 3 shows a joint diagram illustrating the kinematics of a movable arm part of a surgical arm for use in minimally invasive surgery;
Fig. 4 shows a joint diagram illustrating the kinematics of a motion controller which may enable a user to control the surgical instrument;
Fig. 5 shows a surgical instrument inserted into an eye via a trocar;
Figs. 6A-6F show different steps of a subretinal injection of substance below a retinal surface with a surgical instrument which comprises a needle, in which:

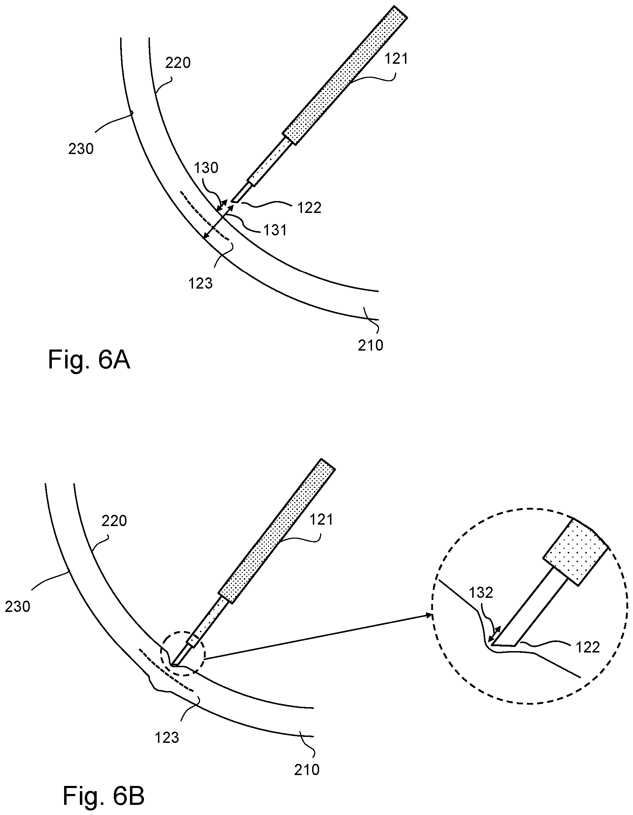

   Fig. 6A shows the needle after approaching the inner retinal surface;
   Fig. 6B shows the needle indenting the inner retinal surface;

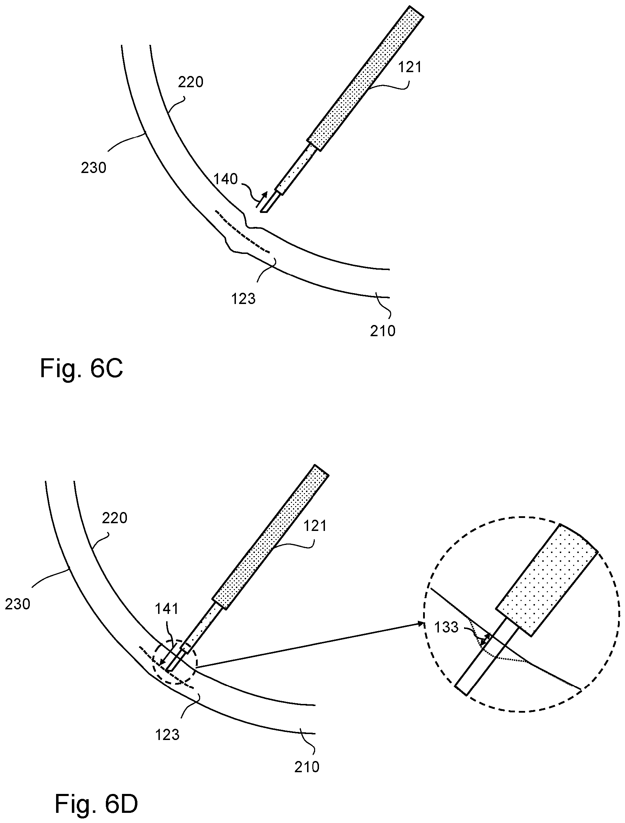

   Fig. 6C shows the needle retracting from the indented location;
   Fig. 6D shows the needle advancing to pierce the inner retinal surface;

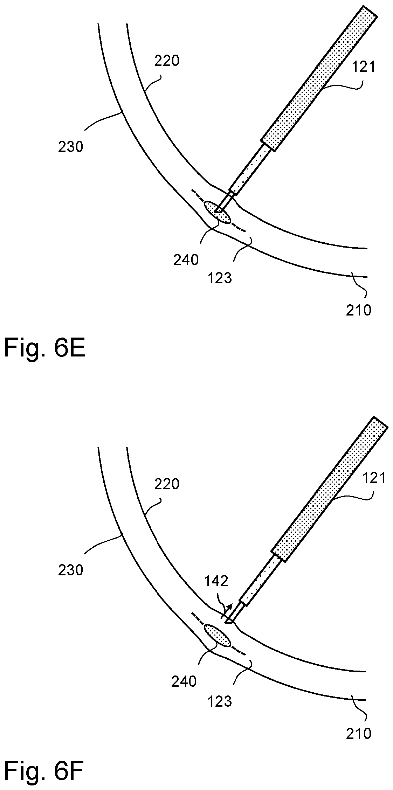

   Fig. 6E shows substance delivered via the needle, forming a bleb;
   Fig. 6F shows the needle withdrawing from the injection site;

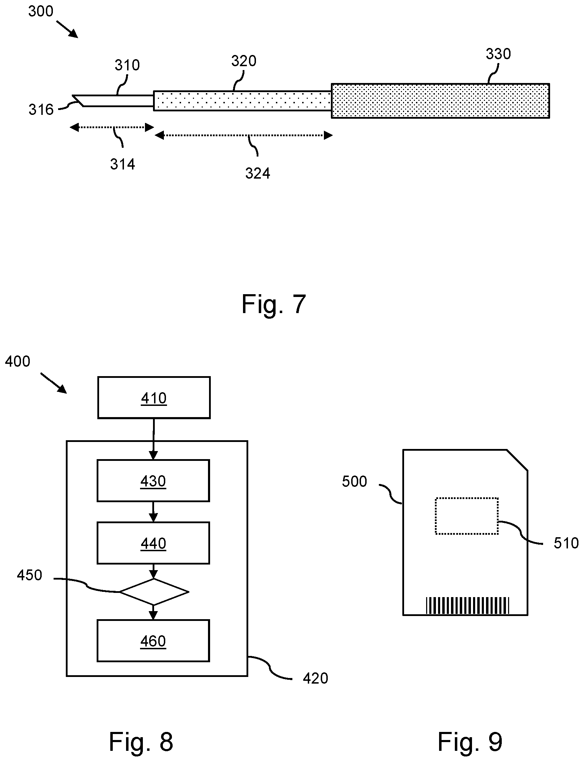

   Fig. 7 shows an example of a needle for the subretinal injection;
   Fig. 8 schematically shows a method for controlling a surgical robotic system during a surgical procedure; and
   Fig. 9 shows a non-transitory computer-readable medium;

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of references and abbreviations is provided for facilitating the interpretation of the drawings and shall not be construed as limiting the claims.
- 20: user interface subsystem
- 22: user inputs
- 30: sensor
- 32: sensor data
- 40: processor subsystem
- 42: actuation commands
- 60: actuator subsystem
- 62: actuation of surgical arm
- 80: surgical arm
- 82: movable arm part
- 100: surgical robotic system
- 104: *̅e̅*̅ₓ, axis of coordinate system fixed to the instrument tip, orthogonal to the instrument longitudinal axis
- 105: *e̅_{y},* axis of coordinate system fixed to the instrument tip, orthogonal to the instrument longitudinal axis
- 106: *̅e̅*̅_{z}, axis of a cartesian coordinate system, aligned with the instrument longitudinal axis
- 107: *ϕ*, rotation of surgical instrument, laterally displacing its tip
- 108: *ψ*, rotation of surgical instrument, laterally displacing its tip
- 109: *z*, longitudinal (along its longitudinal axis) translation of surgical instrument, or penetration direction, or advancing direction
- 110: θ, rotation of surgical instrument around its longitudinal axis
- 111: *φ*, rotational DoF of a movable arm part
- 112: *ψ*, rotational DoF of a movable arm part
- 113: *z*, translational DoF of a movable arm part
- 114: *θ*, rotational DoF of a movable arm part
- 115: *φₘ*, rotational DoF of motion controller
- 116: *ψₘ*, rotational DoF of motion controller
- 117: *zₘ*, translational DoF of motion controller
- 118: *θ*ₘ, rotational DoF of motion controller
- 119, 120: surgical instrument
- 121: needle
- 122: needle tip
- 123: target depth
- 124: trocar
- 125: remote centre of motion (RCM)
- 126: button on motion controller gripper

- 130: distance to inner retinal surface
- 131: distance to outer retinal surface
- 132: indentation depth before penetration
- 133: indentation depth after penetration

- 140: retraction movement
- 141: advancing movement
- 142: withdrawing movement

- 200: eye
- 210: retina
- 220: first layer of tissue (inner retinal surface)
- 230: second layer of tissue (outer retinal surface)
- 240: substance bleb

- 300: concentric assembly
- 310: inner tube
- 314: inner tube protrusion length
- 316: bevelled tip
- 320: intermediate tube
- 324: intermediate tube protrusion length
- 330: outer tube

- 400: method of controlling a surgical robotic system
- 410: controlling actuator subsystem
- 420: performing (part of) subretinal injection procedure
- 430: indenting retinal surface with needle
- 440: performing piercing movement with needle
- 450: determining quality of penetration
- 460: delivering substance via needle

- 500: non-transitory computer readable medium
- 510: data representing computer program

### DESCRIPTION OF EMBODIMENTS

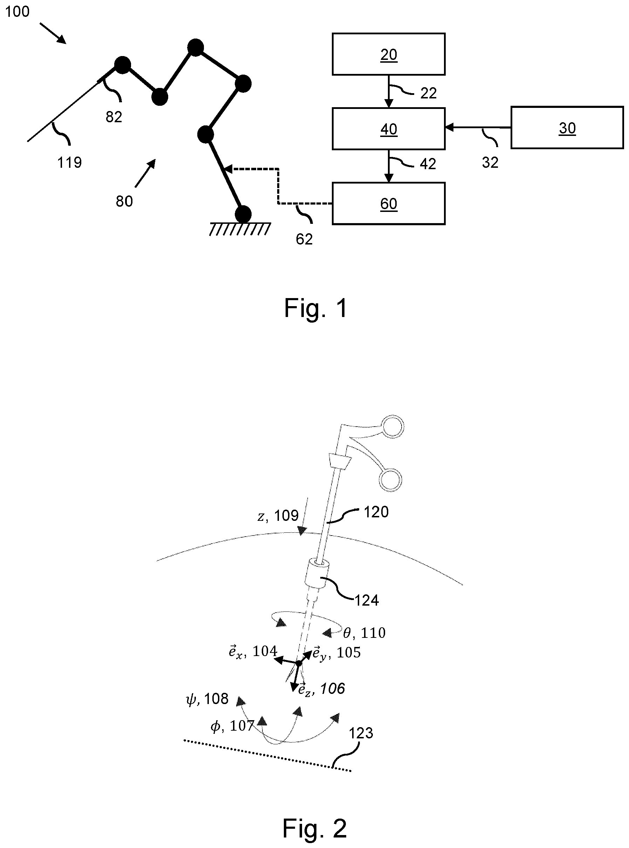

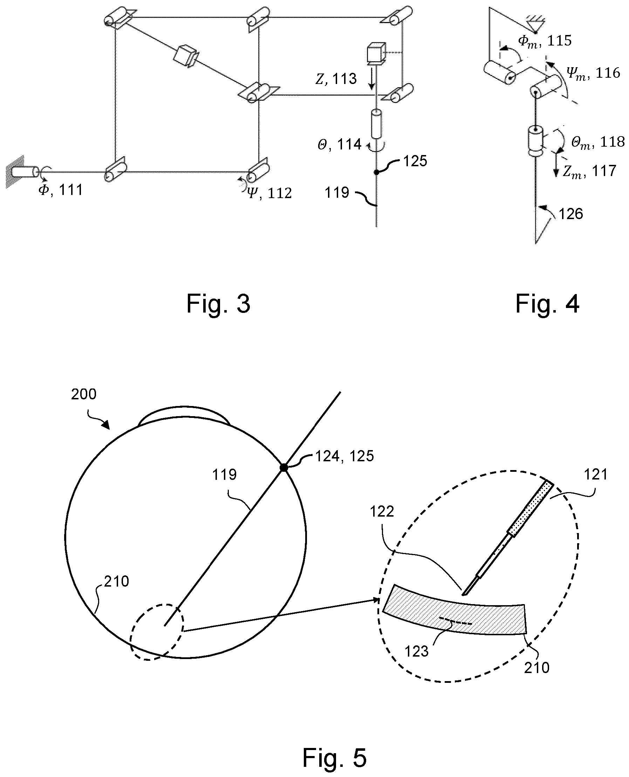

The following embodiments pertain to a surgical robotic system designed for use in a surgical procedure. During such a procedure, the surgical robotic system may perform at least part of a subretinal injection procedure by controlling the pose of a surgical instrument that includes a needle for subretinal injection of a substance. Steps of the injection procedure are detailed with reference to Figs. 5-6F, while Fig. 7 illustrates an example of a needle. Additionally, Figs. 1-4 provide details of the surgical robotic system. However, the embodiments described in this specification are not limited to this specific type of surgical robotic system. Instead, these embodiments may be adapted and applied to other surgical robotic systems for use in intraocular procedures. It is further noted that the figures are schematic representations, and dimensions shown, such as lengths, thicknesses, and depths, are not drawn to scale.

**Fig. 1** schematically shows a surgical robotic system 100. The surgical robotic system 100 may comprise a surgical arm 80. The surgical arm 80 may comprise a movable arm part 82 which may comprise an end effector (not explicitly shown in Fig. 1) for holding a surgical instrument 119. The surgical instrument 119 may comprise, and in some cases be, a needle designed for subretinal injection of a substance below a retinal surface. The needle may for example be an SRI needle. To hold the surgical instrument 119, the end effector may comprise an instrument connector (not explicitly shown in Fig. 1) to which the surgical instrument 119 may be mounted. The movable arm part 82 may have three or more degrees of freedom (DoF) to enable longitudinal movement of the surgical instrument towards a surgical target within the eye and lateral movement of the surgical instrument in a plane normal to said longitudinal movement. Here, longitudinal movement may refer to a movement of the surgical instrument 119 along its longitudinal axis. In some embodiment, the surgical robotic system 100 may be configured to enable lateral movement in a curved plane. The curvature of the curved plane may for example follow a curvature of the interior of the eye. Such type of lateral movement along a curved plane may be obtained by the surgical robotic system adjusting the longitudinal position of the surgical instrument 119 during lateral movement. The shape of the curved plane may for example be determined based on a model of the eye or an intraocular structure, e.g., the retina.

In some embodiments, the surgical robotic system 100 may further comprise a user interface subsystem 20 for receiving user inputs 22 from a user. The user interface subsystem 20 may thus represent at least a user input interface, and in some embodiments, as also discussed elsewhere, additionally or alternatively a user output interface. The user inputs 22 may for example comprise positioning instructions from a human operator, e.g., a surgeon, to enable the human operator to determine a trajectory of the surgical instrument. In some embodiments, the positioning instructions may be positioning commands for directly controlling the movement of the surgical instrument. In other embodiments, the positioning instructions may be provided as input during a planning procedure in which the trajectory of the surgical instrument is planned. Other types of user inputs may for example include confirmation inputs indicating instructions to continue with a procedure or procedural step, and/or input information, such as indicating an operating parameter and/or an indication of a surgical target position or the like. Examples of user input interfaces for receiving user inputs from a user include, but are not limited to, a keyboard, a mouse, a touch-sensitive surface, a joystick, a foot pedal, a microphone, a gesture recognition system, etc. The user input interface may employ any suitable input modality, such as touch, push-actions, voice commands, eye movements, gesture recognition, etc.

The surgical robotic system 100 may further comprise an actuator subsystem 60 configured and arranged for actuating the movable arm part to effect the longitudinal movement and lateral movement of the surgical instrument. The actuator subsystem 60 may comprise any suitable actuator(s), e.g., from the field of surgical robots, or from the more general field of actuators. In particular, the actuator subsystem 60 may comprise a plurality of actuators which together provide the actuation of the movable arm part 60 along the three or more DoFs. Accordingly, it will be appreciated that any reference to a specific configuration of the actuator subsystem 60 may be understood as referring to a (joint) configuration of such a plurality of actuators.

Fig. 1 shows the actuation of surgical arm 80 schematically, namely as a dashed line 62. It is noted that, although shown separately of the surgical arm 80, the actuator subsystem 60 may be integrated into, or mounted to, the surgical arm 80.

In some embodiments, the surgical robotic system 100 may further comprise a sensor 30. The sensor 30 may be or include an optical coherence tomography (OCT) probe. The OCT probe may, for example, be an optical fibre attached to or integrated into the surgical instrument 119, with the optical fibre connected to a remotely located OCT sensor. In other examples, the OCT probe may include an OCT sensor directly attached to or integrated into the surgical instrument 119. The sensor data 32 provided by the OCT probe may include A-scans comprised of line measurements defined as intensity as a function of distance, B-scans forming a 2D image, C-scans derived from multiple B-scans, or similar data types. It is noted that, although the sensor 30 is shown in Fig. 1 as separate from the surgical instrument 119, the sensor or part thereof may be attached to or integrated into the surgical instrument 119. In other examples, the sensor 30 may, in addition to or as an alternative to the OCT probe, include an extraocular OCT sensor located outside the eye but configured to intraoperatively acquire sensor data of the interior structures of the eye. For example, the OCT sensor may be integrated into a microscope communicatively coupled to the surgical robotic system 100. It is further noted that instead of including the sensor 30 directly, the surgical robotic system 100 may comprise a sensor data interface to a sensor which itself may be external to the surgical robotic system.

The surgical robotic system 100 may further comprise a processor subsystem 40 configured to control the actuator subsystem 60 to control at least the longitudinal positioning of the surgical instrument 119 during the intraocular procedure. For this purpose, the processor subsystem 40 may provide actuation commands 42 to the actuator subsystem 60. Specifically, the processor subsystem 40 may be configured to control the actuator subsystem 60 to perform at least part of a subretinal injection procedure, which may comprise the processor subsystem 40 controlling at least a longitudinal positioning of the surgical instrument 119 to indent the retinal surface with the tip of the needle, and from the indented state of the retinal surface, performing a piercing movement. The piercing movement may comprises advancing the surgical instrument longitudinally by a forward distance sufficient to penetrate the retinal surface and reach a target depth below the retinal surface. These and other steps of the injection procedure will be further explained with reference to Figs. 6A-6F.

In general, certain steps of the subretinal injection procedure may be carried out automatically by the surgical robotic system, without requiring the human operator (also referred to as the "user") to directly steer or manually control the robot during those steps. Nevertheless, it is appreciated that the human operator may remain involved in the procedure, for example by providing confirmation between individual surgical steps, as also discussed with reference to optional features and embodiments. In some embodiments, the processor subsystem 40 may operate in an operator control mode, in which it directly executes positioning commands received from the human operator. The procedural steps carried out by the surgical robotic system may be performed while operating in this operator control mode, or after temporarily pausing such an operator control mode. Alternatively, or additionally, the processor subsystem 40 may operate in an autonomous control mode, in which it autonomously controls the position of the surgical instrument, or in a semi-autonomous control mode which combines elements of both the operator control mode and the autonomous control mode. The procedural steps carried out by the surgical robotic system may be performed while operating in either autonomous or semi-autonomous modes.

**Fig.** 2 illustrates the DoFs of a surgical instrument 120 mounted to a surgical robotic system of the type shown in Fig. 1. The surgical instrument 120, which in this example is depicted as a forceps but could equally represent a surgical instrument comprising a needle, is shown passing through a trocar 124 to reach the interior of the eye during an intraocular procedure. For example, in case of vitreoretinal surgery, the trocar 124 may be placed in the sclera. The surgical instrument 120 may be controlled to rotate around and translate through the trocar in four DoF, e.g., the rotations *ϕ* 107, *ψ* 108, *θ* 110 and the translation *z* 109. Further shown are three axes 104-106 of a coordinate system fixed to the instrument tip, e.g., the tip of the needle, with *̅e̅*̅_{z} 106 aligned with the longitudinal axis of the surgical instrument 120. Rotations *ϕ* 107 and *ψ* 108 may result in a lateral displacement of the instrument tip, respectively in the direction *e̅_{y}* 105 and in the direction *e̅ₓ* 104. The translation z 109 may result in a longitudinal movement of the surgical instrument tip. Through such DoFs, the surgical robotic system may control the surgical instrument to position the tip of the needle at an intended penetration site, to control the angle of approach to the penetration site, and to pierce the retina to reach a target depth 123 below the retinal surface.

**Fig. 3** shows a joint diagram illustrating the kinematics of a movable arm part of a surgical robotic system of the type shown in Fig. 1. The movable arm part may have DoFs *Φ* 111, *ψ* 112, Z 113 and *Θ* 114 to allow instrument motions 107-110 as previously shown in Fig. 2 to adjust the pose of the surgical instrument 119 and its tip. The DoFs may be arranged such that there is a point on the surgical instrument 119 that does not move in space, which point may also be referred to as remote centre of motion (RCM) 125. By moving the base of the surgical arm, the movable arm part may be positioned such that the RCM 125 is positioned at the trocar. Respective actuators may be arranged to effect movement in all four DoFs 111-114.

As previously discussed with reference to Fig. 1, the surgical robotic system may comprise a user input interface for receiving user inputs, such as positioning instructions, from a human operator, e.g., a clinical user such as a surgeon. In some embodiments, the user interface may comprise or be constituted by a motion controller such as a joystick. In some embodiments, the motion controller may be an external device with which the user interface or the processor subsystem 40 is configured to interface. In some embodiments, the motion controller may be comprised in the surgical robotic system as a further component, e.g., configured to interface with the processor subsystem 40 and/or the user interface subsystem 20. **Fig. 4** shows a joint diagram illustrating the kinematics of such a motion controller. Here, the motion controller is shown to have DoFs *Φₘ* 115, *ψₘ* 116, *Zₘ* 117 and *Θₘ* 118. The user may provide user inputs, such as positioning commands to directly control the movement of the movable arm part, for example by holding the motion controller at a gripper part, pressing the button 126 and moving the gripper part of the motion controller in space.

**Fig. 5** shows a surgical instrument 119 positioned within an eye 200. As elucidated elsewhere, the surgical instrument 119 may comprise, or be, a needle 121. As shown on the left-hand side of Fig. 5, the surgical instrument 119 may be inserted into the eye 200, for example, through a trocar 124, with the remote centre of motion (RCM) 125 of the surgical instrument co-located with the trocar 124. The surgical instrument 119 is depicted as approaching the retina 210. On the right-hand side of Fig. 5, a magnified view of the needle tip 122 is shown. In this example, the surgical instrument 119 may be controlled to pierce the retina 210 to reach a target depth 123 below the inner retinal surface. It will be appreciated that while Fig. 5 and subsequent figures illustrate the target depth 123 for ease of illustration as being substantially in the middle of the retina 210, for subretinal injections, the target depth may generally lie within the subretinal space, e.g., at the outer boundary of the retina, between the neurosensory retina and the retinal pigment epithelium (RPE). In order to deliver the substance to be injected, the tip 122 of the needle may comprise an injection lumen. Although not explicitly illustrated in Fig. 5, a sensor or sensor component, such as an optical fibre, may be integrated into or attached to the surgical instrument 119. For example, the sensor may include an optical fibre coupled to an OCT sensor, configured to emit an optical beam and capture cross-sectional images of the retina 210 and other ocular structures. The optical fibre may be recessed from the needle tip 122 of the surgical instrument 119, allowing the OCT sensor to capture sensor data even while the needle tip is piercing or penetrating a tissue layer. Using the OCT probe, the surgical robotic system may obtain sensor data indicative of a reflectivity depth profile. The reflectivity depth profile may provide information about the thickness of the retina and may aid in the precise positioning of the tip 122 relative to the retinal surface. This may enable controlled indentation of the retina or, after indentation, accurate placement of the needle tip at the target depth 123. Additionally, such sensor data may help assess the quality of penetration before starting substance delivery.

While the above refers to the use of an OCT sensor, it is noted that additionally or alternatively, other intraocular sensing modalities may be used, such as Ultrasound or touch. Moreover, as previously explained with reference to Fig. 1, extraocular sensor data may be used in addition to or as an alternative to the use of intraocular sensor data obtained from an OCT probe. For example, extraocular sensor data may be obtained from an extraocular OCT sensor, or from Ultrasound.

Figs. 6A-6F illustrate different steps of a subretinal injection of substance below a retinal surface using a surgical instrument comprising a needle. It is noted that a surgical robotic system may automate any subset of these steps or all of the steps. Exemplary combinations of steps are indicated by the claims and their dependencies. Notwithstanding such automation, the user may retain a degree of control over the subretinal injection of the substance, for example, by being required to confirm progression to a subsequent step and by being able to abort the procedure. Such confirmation may for example be provided via a user interface as described elsewhere in this specification and may rely on feedback presented to the user, e.g., sensor data showing the penetration site or measurement data. Confirmation may also be required for only a subset of steps, for example those deemed critical, such as proceeding with the piercing movement or initiating the injection of substance, or when automated assessment of the quality of penetration indicates uncertainty on whether to proceed.

**Fig. 6A** shows the needle 121 with its needle tip 122 positioned in proximity to the retina 210. The retina 210 is shown to include an inner retinal surface 220, e.g., facing the vitreous cavity, and an outer retinal surface 230. The needle tip 122 is depicted at a distance 130 from the inner retinal surface 220. This distance 130 may for example be measured using an OCT sensor. The OCT sensor may for example be an intraocular or extraocular sensor as described elsewhere in this specification. The needle 121 may be positioned by a surgical robotic system based on the measurements of the distance 130 to enable the needle tip 122 to be precisely positioned with respect to the retina 210. Fig. 6A also shows the distance 131 from the needle tip 122 to the outer retinal surface 230. This distance 131 may likewise be measured using an OCT sensor. Both distances 130 and 131 may be used to determine the thickness of the retina 210. This thickness, which may vary between patients and locations on the retina, may allow determining a target depth 123, which may be the depth below the inner retinal surface 220 for subretinal injection of substance. While the target depth may be defined in relation to the inner retinal surface 220, the target depth may generally lie in the subretinal space, e.g., between the neurosensory retina and the retinal pigment epithelium (RPE).

**Fig. 6B** shows the needle 121 with its needle tip 122 indenting the outer retinal surface 220 of the retina 210. Before initiating the indentation, the surgical robotic system may first position the needle tip 122 so that it contacts, e.g., touches, the outer retinal surface 220. This step of establishing contact with the retina 210 may provide a controlled starting point for the indentation process. The touch of the needle tip 122 on the outer retinal surface 220 may be verified using intraoperative sensor data. For example, an intraocular or extraocular OCT sensor may be used to detect and confirm the contact. Alternatively, other modalities, such as a touch sensor or force sensor integrated into the surgical instrument, may be used to confirm the contact. Once the contact is confirmed, the surgical robotic system may control the needle 121 to advance longitudinally to achieve a specific indentation depth 132. It is noted that such indentation may be optional, since in some cases, the indentation step may be omitted to directly proceed with a piercing movement from the needle tip 122 touching the outer retinal surface 220. The longitudinal movement for the indentation may be controlled and relatively slow, for example 0,5-1 mm/s, so that the outer retinal surface 220 is indented with precision and not damaged. The indentation depth 132 may for example fall within a range of 20 to 150 µm relative to the outer retinal surface 220. Following the indentation, the surgical robotic system may confirm that the outer retinal surface 220 has achieved the indented state. This confirmation may be based on intraoperative sensor data indicative of the indentation or may be obtained through a user input provided via a user interface. Such confirmation may be required before the surgical robotic system proceeds to subsequent steps, such as initiating a piercing movement as shown in Figs. 6C, 6D.

**Fig. 6C** shows a first step of a piercing movement performed by the needle 121 with its needle tip 122. This movement may directly follow the indentation step as shown in Fig. 6B. The piercing movement may generally involve advancing the needle 121 longitudinally by a forward distance to penetrate the outer retinal surface 220 and reach the target depth 123 below the retinal surface. However, Fig. 6C illustrates an optional step where the processor subsystem is configured to control the surgical instrument to first perform a retraction movement. In this step, the needle 121 may be retracted longitudinally by a retraction distance. This retraction movement 140 may serve as a preparatory step to position the needle tip 122 for advancement.

**Fig. 6D** shows an advancing step of the piercing movement, in which the needle 121 is advanced longitudinally to pierce the outer retinal surface 220. This advancing movement 141 may achieve penetration of the outer retinal surface 220 and position the needle tip 122 at the target depth 123. When the advancing movement 141 is preceded by a retraction step, the needle 121 may be advanced longitudinally by a forward distance that may exceed the retraction distance, with both distances being selected to jointly achieve the penetration until the target depth 123 is reached. In some embodiments, the forward distance may be selected as approximately twice the retraction distance. The advancement, and the optional preceding retraction, may be carried out immediately following the indentation, ensuring a continuous and controlled sequence of movements. In this manner, the surgical robotic system may position the needle tip 122 within the retina 210 at the target depth 123, which may for example lie between 150 and 250 µm below the outer retinal surface 220. The processor subsystem may perform the advancement within a time during which the indentation of the outer retinal surface 220 is still in the process of restoring. This time between initiating the retraction step and having carried out the advancement step may for example be less than 50 ms, less than 40 ms, less than 30 ms, less than 25 ms, less than 20 ms, less than 15 ms, or less than 10 ms.

In some embodiments, following the piercing movement, the processor subsystem may assess the quality of penetration by analysing intraoperative sensor data of the penetration site. For example, the retina 210 may exhibit an indentation at the penetration site, which indentation may be characterized by an indentation depth 133. This indentation depth may be used as a parameter to evaluate the penetration quality. Specifically, if the indentation depth 133 exceeds a predefined threshold, the penetration may be deemed unsatisfactory in that backflow of substance into the vitreous chamber may be likely. In some embodiments, the absence of indentation may be identified by verifying the curvature of the outer retinal surface 220. If the outer retinal surface 220 does not exhibit a constant curvature, the surgical robotic system may refrain from automatically advancing to the next step, such as substance delivery shown in Fig. 6E. It is noted that such quality assessment may be implemented in various ways, for example by employing image analysis techniques based on heuristics or by making use of machine learning methods. For example, a machine learning model may be trained to provide an assessment of penetration quality from intraocular sensor data as input. In a specific example, the machine learning model may be a convolutional neural network (CNN) or Vision Transformer (ViT)-based neural network (NN) which may be trained for a regression task so as to provide a continuous numerical value indicative of the penetration quality as output. In other examples, the machine learning model, such as the aforementioned CNN or ViT-based NN, may be trained to perform a classification task in which the class labels correspond to different penetration quality levels. Alternatively, the assessment of penetration quality may rely partially or entirely on user input. For example, the user may be involved in the assessment if the surgical robotic system determines that the indentation depth 133 exceeds the threshold or, alternatively, the user may be involved instead of the surgical robotic system performing an automated assessment of penetration quality.

**Fig. 6E** shows substance being delivered via the needle 121 after the needle tip 122 is positioned at the target depth 123 below the outer retinal surface 220. As the substance is injected, a substance bleb 240 may be formed within the retina 210. In some embodiments, the injection process may be controlled by the surgical robotic system. For that purpose, the surgical robotic system may include a pump control interface operatively connected to a pump configured to deliver the substance through the needle 121, e.g., through an injection lumen of the needle 121. The processor subsystem may for example regulate the injection process by controlling the pump to adjust the injection volume and flow rate. For example, the volumetric flow rate may be gradually increased during the subretinal injection procedure to ensure controlled delivery of the substance. The formation of the substance bleb 240 may result from this controlled delivery. The processor subsystem may also be configured to monitor the injection volume in real time based on intraoperative sensor data. This monitoring may enable the surgical robotic system to stop the injection when a predetermined target injection volume has been delivered, preventing over-injection and ensuring that the substance bleb 240 remains within desired parameters.

**Fig. 6F** depicts the needle 121 with its tip 122 withdrawing from the injection site after substance delivery and the formation of the substance bleb 240. The withdrawing movement 142 may be regulated by the processor subsystem to enable gradual retraction of the needle 121 from the retina 210. The withdrawal may for example be performed in a stepwise manner, such as in increments of 100 µm. The processor subsystem may pause between retraction steps. Such pauses may allow the tissue to settle if it is observed to be pulled upward with the needle. A pause may enable the tissue to slowly return to its original position, reducing tension and reducing the risk of creating or enlarging an opening or wound. This approach may facilitate proper closure of the retinotomy and promote better tissue healing. In some embodiments, the processor subsystem may pause following the completion of the substance delivery and before initiating the withdrawing movement 142. This pause may allow for stabilization of the injection site and the substance bleb 240 created during the substance delivery. After the pause, the processor subsystem may proceed with the withdrawal, for example using the stepwise retraction described above.

**Fig. 7** shows an exemplary needle which may be used by the surgical robotic system in the subretinal injection procedure. The needle may comprise a concentric assembly 300 of tubes 310, 320, 330. Fig. 7 shows a side view of a distal section of the concentric assembly 300. The distal section of the concentric assembly 100 may enter a patient's eye during surgery. The needle may further comprise a proximate section (not shown in Fig. 7) which may remain outside of the eye during surgery. For example, the proximate section of the needle may comprise a handle to enable the needle to be manipulated, e.g., by the surgical robotic system or by any other mechanism designed to support manipulation of the needle. In this respect, it is noted that the terms 'distal' and 'proximate' may assume an operator of the needle as a point of reference, meaning that a distal section or end may be located away from the operator and typically nearest to a surgical target, while a proximate section or end may be located towards the operator and typically farthest from the surgical target.

The concentric assembly 300, which may extend in longitudinal direction beyond the length shown in Fig. 7, may comprise an outer tube 330 and an inner tube 310 concentrically arranged within the outer tube 330. The inner tube 310 may comprise a tip 316 for piercing a retinal surface layer, with the tip 316 being arranged at a distal end of the inner tube 310. The concentric assembly 300 may be arranged to circumferentially taper-down along a longitudinal direction towards the tip 316 from the outer tube 330 to the inner tube 310, meaning that the outer diameter of the concentric assembly 300 may be reduced from an outer diameter of the outer tube 330 via an intermediate outer diameter to an outer diameter of the inner tube 310.

In the example of Fig. 7, this circumferential reduction is provided by the concentric assembly 300 comprising an intermediate tube 320 concentrically arranged between the inner tube 310 and the outer tube 330. While the outer diameter of the inner tube 310 and the outer diameter of the outer tube 330 may be substantially constant in the distal section of the concentric assembly 300 or along the length of the concentric assembly 300, the intermediate tube 320 may provide an intermediate outer diameter and thereby a stepwise reduction in outer diameter from the outer diameter of the outer tube 330 to the outer diameter of the inner tube 310. It will be appreciated that in addition or alternatively to use of an intermediate tube 320, the outer tube 330 and/or the inner tube 310 may be tapered so that a respective tube comprises an outer diameter which varies over a range which includes the intermediate outer diameter.

Fig. 7 further shows the intermediate tube 320 extending axially from a distal end of the outer tube 330 and the inner tube 310 extending axially from a distal end of the intermediate tube 320. For example, the inner tube 310 may extend axially from the distal end of the intermediate tube 320 by a length 314 of 1 mm or less, or by 750 µm or less, or by 500 µm ± 100 µm, or by 500 µm, while the intermediate tube 320 may extend axially from the distal end of the outer tube 330 by a length 324 of 1 mm to 3 mm, or by a length of 2 mm ± 500 µm, or by 2 mm ± 250 µm, or by 2 mm. It is noted that by extending axially outward from the intermediate tube 320, the inner tube 310 also extends axially outward from the outer tube, e.g., by a greater length. It will be further appreciated that Fig. 7 is not drawn to scale and that any dimensions shown in the figures may not be representative of dimensions as described.

Fig. 7 shows the tip 316 of the inner tube 310 as being bevelled. The bevel angle may for example be 45° ± 10°, or 45° ± 5°, or 45° ± 2.5°, or 45°.

The concentric assembly 300 may form a lumen for injecting a substance. Specifically, the lumen may be formed by the interior walls of the respective tubes 310, 320, 330. The lumen may comprise a first opening at the tip 316 of the inner tube 310, with the first opening being elsewhere also referred to as an exit aperture, and a second opening at a proximate section of the needle (not shown in Fig. 7). The substance may be supplied via the second opening, for example by a pump, and then delivered through the lumen within the needle to the tip 316, where the substance may be expelled from the first opening and thereby injected into retinal tissue.

In some examples, the tubes 310, 320, 330 may have outer diameters which may be substantially constant along the lengths of the respective tubes, while in other examples, one or more of the outer diameters may vary along the length of a respective tube. In some examples, the outer diameter of the inner tube 310 may be selected to substantially match an inner diameter of the intermediate tube 320, and the outer diameter of the intermediate tube 320 may be selected to substantially match an inner diameter of the outer tube 330. This may facilitate attachment of the inner tube 310 to an inside of the intermediate tube 320 and attachment of the intermediate tube 320 to an inside of the outer tube 330. For example, the inner tube 310 may be affixed with an adhesive, such as glue, to the inside of the intermediate tube 320 and the intermediate tube 320 may be affixed with an adhesive, such as glue, to the inside of the outer tube 330. Other examples to attach respective tubes to each other may be interference fitting or press fitting of respective tubes, heat shrinking, ultrasonic welding, etc. With continued reference to the outer diameter of the inner tube 310, the outer diameter may be selected to be between 80-160 µm at an onset of the bevel, or 120 µm or less, or 110 µm or less, or 100 µm or less, or 80 µm ± 10 µm, or 80 µm. With continued reference to the outer diameter of the intermediate tube 320, the outer diameter may be between 140 µm and 180 µm, for example 160 µm ± 10 µm or 160 µm.

The inner tube 310 of the concentric assembly 300 may comprise or may be made of a material which is at least partially translucent to OCT. For example, the inner tube 310 may comprise or may be made out of a translucent plastic, for example a translucent thermoplastic. In a specific example, the inner tube 310 may comprise or may be made out of a translucent polymer such as polyimide. Materials which may be considered to be at least partially translucent to visible light may provide a sufficient degree of translucency to OCT. Polyimide may be naturally translucent to visible light. The intermediate tube 320 may in some examples also comprise or may be made of a material which is at least partially translucent to OCT. For example, the intermediate tube 320 may comprise or may be made out of a translucent plastic, for example a translucent thermoplastic. In a specific example, the intermediate tube 320 may comprise or may be made out of a translucent polymer such as polyimide. In a specific example, both the inner tube 310 and the intermediate tube 320 may comprise or may be made out of the same material, for example polyimide. In other examples, the intermediate tube 320 may comprise or may be made out of a different material, for example a non-translucent material such as metal. An example of a metal may be stainless steel, e.g., AISI 304 or AISI 316. The outer tube 330 may comprise or may be made out of metal, for example stainless steel, e.g., AISI 304 or AISI 316, or any material known to provide sufficient structural strength in a needle application.

In general, the needle may be a needle for subretinal injection as described in EP 24186591, which hereby incorporated by reference in its entirety or at least in as far as describing the needle including any of its dimensions and material properties.

In general, any image analysis performed by the processor subsystem may be based on heuristically designed image analysis techniques or machine learning methods. Exemplary machine learning approaches may include training a neural network for tasks such as image classification or regression analysis. Examples of neural networks that may be utilized include, but are not limited to, convolutional neural networks (CNNs) and Vision Transformer (ViT)-based neural networks. The processor subsystem may access model data corresponding to a trained machine learning model and use this model during the intraocular procedure for inference, such as for the aforementioned classification or regression analysis of intraocular sensor data.

In general, the processor subsystem described in this specification may comprise one or more (micro)processors which execute appropriate software. The software implementing the functionality of the processor subsystem may have been downloaded and/or stored in a corresponding memory or memories, e.g., in volatile memory such as RAM or in non-volatile memory such as Flash. Alternatively, the processor subsystem may be implemented in the form of programmable logic, e.g., as a Field-Programmable Gate Array (FPGA). Any input and/or output interfaces may be implemented by respective hardware and/or software interfaces. In general, the processor subsystem, input interfaces, and/or output interfaces may each be implemented in the form of a circuit or circuitry. The processor subsystem may also be implemented in a distributed manner, e.g., involving different devices or apparatus.

**Fig. 8** shows a method 400 of controlling a surgical robotic system during a surgical procedure. The method 400 may comprise controlling 410 an actuator subsystem to control a position of the surgical instrument during a surgical procedure, and simultaneously or alternatingly in time with said controlling, performing 420 at least part of a subretinal injection procedure, which may comprise indenting 430 a retinal surface with a needle, performing 440 a piercing movement with the needle, and as optional steps, determining 450 a quality of penetration of the retina, and if the quality of penetration is deemed satisfactory, continuing with delivering 460 a substance via the needle. The aforementioned steps 420-460 may correspond to corresponding steps described elsewhere in this specification for the surgical robotic system.

It is noted that any of the methods described in this specification, for example in any of the claims, may be implemented on a computer as a computer implemented method, as dedicated hardware, or as a combination of both. Instructions for the computer, e.g., executable code, may be stored as a computer program on a computer-readable medium 500 as for example shown in **Fig. 9****,** e.g., in the form of a series 510 of machine-readable physical marks and/or as a series of elements having different electrical, e.g., magnetic, or optical properties or values. The executable code may be stored in a transitory or non-transitory manner. Examples of computer-readable mediums include memory devices, optical storage devices, integrated circuits, servers, online software, etc. Fig. 9 shows by way of example a memory card 500.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A surgical robotic system (100) for use in an intraocular procedure, comprising:
- a surgical arm (80) comprising a movable arm part (82), wherein the movable arm part comprises an end effector for holding a surgical instrument (119), wherein the surgical instrument comprises a needle (121) for subretinal injection of a substance below a retinal surface (220);
- an actuator subsystem (60) configured to actuate the movable arm part;
- a processor subsystem (40) configured to control the actuator subsystem to control a longitudinal positioning of the surgical instrument during the intraocular procedure, wherein the processor subsystem is configured to control the actuator subsystem to perform at least part of a subretinal injection procedure by:
- with a tip (122) of the needle, indent the retinal surface (220); and
- from said indented state of the retinal surface, perform a piercing movement (141), wherein the piercing movement comprises advancing the surgical instrument longitudinally by a forward distance to penetrate the retinal surface and reach a target depth (123) below the retinal surface.

2. The surgical robotic system (100) according to claim 1, wherein the processor subsystem (40) is configured to control the actuator subsystem (60) to indent the retinal surface (220) by:
- positioning the tip (122) of the needle to touch the retinal surface;
- accessing intraoperative sensor data (32) to detect and confirm the touch of the retinal surface; and
- upon confirmation of the touch, advancing the needle longitudinally by an indentation distance (132), for example 20-150 µm, to achieve the indented state of the retinal surface.

3. The surgical robotic system (100) according to claim 1 or 2, wherein the processor subsystem (40) is further configured to confirm the indented state of the retinal surface (220) before initiating the piercing movement, wherein said confirmation comprises receiving a user input (22) indicative of the indented state or analysing intraoperative sensor data (32) indicative of said indentation of the retinal surface.

4. The surgical robotic system (100) according to any one of claims 1 to 3, wherein the processor subsystem (40) is further configured to:
- obtain an assessment of a quality of penetration of the retinal surface after the piercing movement; and
- based on the assessment, either continue or abort the subretinal injection procedure, wherein continuing comprises controlling the surgical instrument (119) to perform the subretinal injection of the substance via the needle (121).

5. The surgical robotic system (100) according to claim 4, further comprising a sensor interface configured to access intraoperative sensor data (32) indicative of a shape of the retinal surface after penetration, wherein the processor subsystem (40) is further configured to assess the quality of penetration by:
- analysing a shape of the retinal surface (220) at a penetration site based on the intraoperative sensor data; and
- determining that the quality of penetration is unsatisfactory if the retinal surface exhibits an indentation (133) at the penetration site which exceeds a predefined indentation threshold.

6. The surgical robotic system (100) according to claim 4, wherein the assessment of the quality of penetration is obtained at least in part through user input (22) indicative of an evaluation of the penetration.

7. The surgical robotic system (100) according to any one of claims 1 to 6, further comprising a pump control interface operatively connected to a pump configured to deliver the substance for injection via the needle (121), wherein the processor subsystem (40) is further configured to control the pump to regulate an injection volume during the subretinal injection procedure, for example by gradually increasing a volumetric flow rate.

8. The surgical robotic system (100) according to claim 7, wherein the processor subsystem (40) is further configured to:
- monitor the injection volume based on intraoperative sensor data (32); and
- stop the injection of substance when a target injection volume has been delivered.

9. The surgical robotic system (100) according to any one of claims 1 to 8, wherein the processor subsystem (40) is further configured to retract the needle (121) in a stepwise manner after the subretinal injection of the substance, for example in increments of 100 µm.

10. The surgical robotic system (100) according to claim 9, wherein the processor subsystem (40) is further configured to pause after the subretinal injection of the substance and, following the pause, initiate the stepwise retraction of the needle (121).

11. The surgical robotic system (100) according to any one of claims 1 to 10, wherein the processor subsystem (40) is configured to perform the piercing movement by first retracting (140) the surgical instrument (119) longitudinally by a retraction distance, and after the retracting, advancing (141) the surgical instrument longitudinally by a forward distance which exceeds the retraction distance to penetrate the retinal surface and reach the target depth below the retinal surface, wherein preferably the retraction distance and the forward distance are jointly selected to achieve the target depth of 150-250 µm below the retinal surface.

12. The surgical robotic system (100) according to claim 11, wherein the processor subsystem (40) is configured to advance (141) the surgical instrument within a time during which said indentation of the retinal surface (220) is restoring.

13. The surgical robotic system (100) according to any one of claims 1 to 12, wherein the processor subsystem (40) is further configured to determine the target depth (123) intraoperatively based on an angle of approach of the needle and an estimate of a thickness of the retina (210).

14. A computer-implemented method (400) for controlling a surgical robotic system during an intraocular procedure,
wherein the surgical robotic system comprises:
- a surgical arm comprising a movable arm part, wherein the movable arm part comprises an end effector for holding a surgical instrument, wherein the surgical instrument comprises a needle for subretinal injection of a substance below a retinal surface;
- an actuator subsystem configured to actuate the movable arm part;
wherein the method comprises:
- controlling (410) the actuator subsystem to control a longitudinal positioning of the surgical instrument during the intraocular procedure,
wherein said control of the actuator subsystem comprises performing (420) at least part of a subretinal injection procedure by:
- with a tip of the needle, indenting (430) the retinal surface; and
- from said indented state of the retinal surface, performing (440) a piercing movement, wherein the piercing movement comprises advancing the surgical instrument longitudinally by a forward distance to penetrate the retinal surface and reach a target depth below the retinal surface.

15. A computer program (510) comprising instructions which, when executed by a processor, cause the processor to carry out the method of claim 14.
